## Europäisches Patentamt
## European Patent Office
## Office européen des brevets

(19)

(11) Veröffentlichungsnummer: **0 061 710**

**A2**

(12) # EUROPÄISCHE PATENTANMELDUNG

(21) Anmeldenummer: **82102449.4**

(22) Anmeldetag: **24.03.82**

(51) Int. Cl.³: **A 61 K 7/26**
**A 61 K 9/68**

(30) Priorität: **24.03.81 DE 3111448**

(43) Veröffentlichungstag der Anmeldung:
**06.10.82 Patentblatt 82/40**

(84) Benannte Vertragsstaaten:
**CH DE FR GB IT LI NL SE**

(71) Anmelder: **Nachtsheim, Petra**
**Engelsstrasse 19**
**D-5000 Köln 91(DE)**

(71) Anmelder: **Peters, Klaus Michael**
**Burgherrenweg 30**
**D-5060 Bergisch Gladbach(DE)**

(72) Erfinder: **Nachtsheim, Petra**
**Engelsstrasse 19**
**D-5000 Köln 91(DE)**

(72) Erfinder: **Peters, Klaus Michael**
**Burgherrenweg 30**
**D-5060 Bergisch Gladbach(DE)**

(74) Vertreter: **Werner, Hans-Karsten, Dr. et al,**
**Deichmannhaus am Hauptbahnhof**
**D-5000 Köln 1(DE)**

(54) **Kau- und schluckbares Ballaststoffpräparat, Verfahren zu seiner Herstellung und seine Verwendung.**

(57) Kau- und schluckbares Ballaststoffpräparat zur Zahnpflege und Ergänzung der Nahrung wird vorgeschlagen aus überwiegend entzuckerten und entwässerten Zuckerrübenschnitzeln in Dosisform, dem ggf. Bindemittel, Geschmacksstoffe, Aromastoffe, Süßstoffe, fluorhaltige Zusätze, Desinfektionsmittel und Farbstoffe zugesetzt sind. Weiterhin beschrieben wird das Verfahren zur Herstellung solcher Präparate sowie ihre Verwendung zur vorbeugenden Behandlung von Karies und Parodontopathien und Erkrankungen des Gastointestinaltrakts.

## Kau- und schluckbares Ballaststoffpräparat, Verfahren zu seiner Herstellung und seine Verwendung

Gegenstand der vorliegenden Erfindung ist ein kau- und schluckbares Ballaststoffpräparat zur Zahnpflege und Ergänzung der Nahrung sowie Verfahren zu seiner Herstellung und seine Verwendung zur vorbeugenden Behandlung von Karies, Parodontose und Erkrankungen des Gastrointestinaltrakts.

Karies, Parodontose und Erkrankungen des Gastrointestinaltrakts nehmen als typische Zivilisationskrankheiten in erschreckendem Maße zu. Vergleichende Untersuchungen zwischen Naturvölkern, Bevölkerungsgruppen mit herkömmlicher Ernährung und Bevölkerungsgruppen, die zivilisationsbedingt überwiegend schlackenfreie oder schlackenarme hochkonzentrierte, meist noch saccharosehaltige Nahrungsmittel zu sich nehmen, zeigen, daß Karies, Parodontose und Erkrankungen des Gastrointestinaltrakts in erheblichem Maße von den Ernährungsmöglichkeiten und Ernährungsgewohnheiten abhängen. Karies und Parodontose werden bisher mit mehr oder weniger geringem Erfolg durch Zahnpflege mittels Zahnbürsten, Zahncreme und Mundduschen sowie das Kauen von zuckerfreiem Kaugummi bekämpft. Zur Bekämpfung der Karies wird in einigen Teilen der Welt auch das Trinkwasser fluoriert. Fluorhaltige Zusätze zu Zahncreme haben ebenfalls gewisse Erfolge gezeigt. Das Kauen von ballaststoffhaltiger Nahrung wie Äpfeln, Möhren, Zuckerrohr und bestimmten Wurzeln hat sicherlich einen guten vorbeugenden Einfluß auf gewisse Zahnerkrankungen, ist aber aus den verschiedensten Gründen weder für alle Bevölkerungsschichten durchführbar und zumutbar, noch möglich. Außerdem wird der vorbeugende Effekt durch den Zucker- und/oder Säuregehalt dieser Nahrungsmittel herabgesetzt.

Um die ballastarme Ernährungsweise zu kompensieren, wird die zusätzliche Aufnahme von Weizenkleie empfohlen und praktiziert. Weizenkleie ist zwar zur Obstipationsprophylaxe geeignet, kann aber u.U. aufgrund des Bindungsvermögens für Metallionen der in ihr enthaltenden Phytinsäure einen Mangel an Spurenelementen bewirken. Weizenkleie ist aber wegen der fehlenden Kaubeanspruchung für die Zahnpflege ungeeignet. Da Weizenkleie obendrein von den Mundbakterien abbaubare Kohlehydrate enthält, wirkt sie sogar, ähnlich wie Zucker und Stärke,zahnschädigend. Zusammenfassungen über diese Zusammenhänge finden sich in

Sauerwein, Zahnerhaltungskunde, 1972, Thieme-Verlag, Stuttgart, Seiten 85 bis 89,

Royal College of Physicians of London, Medical Aspects of Dietary Fibre, 1980, Pitman Med. Ltd., Seite 124 bis 132 sowie Miller, Bedeutung der Ballaststoffe für die Entstehung und Behandlung gastrointestinaler Erkrankungen; akt. Ernährung 2 (1977, Seite 67).

Nach diesem Stand der Technik läßt sich somit sagen, daß Zahnbürste und Mundduschen zu umständliche Putztechniken sind, die obendrein bei falscher Anwendung zu Schäden an Zähnen und Zahnfleisch führen. Das erforderliche Putzen nach jeder Mahlzeit und an jedem Ort ist für die Mehrzahl der Bevölkerung nicht praktikabel. Mundduschen massieren zwar das Zahnfleisch, ersetzen aber die mechanische Reinigung durch die Zahnbürste nicht. Die für die Erhaltung der Zähne und des Zahnhalteapparates notwendige funktionelle Beanspruchung kann durch diese Pflegemethoden nicht geschaffen werden. Weiterhin fehlt die für die Entstehung von Parodontopathien verantwortliche funktionelle Beanspruchung durch ausreichende Kauarbeit. Kaugummis sind nicht schluckbar, woraus sich das hygienische Problem ergibt, den Kaugummi nach verrichteter Kauarbeit zu beseitigen. Für einen ausreichenden Reinigungseffekt sind 20 Minuten Kaudauer erforderlich. Der angenehme Geschmack der Aroma- und Geschmacksstoffe im Kaugummi ist jedoch meist schon nach ca. 3 Minuten verloren. Die Kauarbeit auf der weichen, nachgiebigen Masse ist weit geringer als beim Kauen von Ballaststoffpräparaten. Das Kauen von Kaugummi kann Sodbrennen und andere Magenbeschwerden hervorrufen.

Die Erfindung hat sich die Aufgabe gestellt, ein kau- und schluckbares Ballaststoffpräparat zur Zahnpflege und Ergänzung der Nahrung zu entwickeln, welches nach Möglichkeit keine der oben aufgezeigten Nachteile aufweist.

Diese Aufgabe wird gelöst durch ein Ballaststoffpräparat, das dadurch gekennzeichnet ist, daß es überwiegend aus entzuckerten und entwässerten Zuckerrübenschnitzeln in Dosisform besteht und gegebenenfalls stärkefreie Bindemittel, Geschmacksstoffe, Aromastoffe, zuckerfreie Süßstoffe, fluorhaltige Zusätze, Desinfektionsmittel und Farbstoffe enthält.

Entzuckerte und entwässerte Zuckerrübenschnitzel sind so wasserarm, daß sie die Speichelsekretion fördern. Da sie praktisch zuckerfrei und frei von anderen abbaubaren Kohlehydraten sind, kann kein Abbau von Zucker durch Mundbakterien zu zahnschädigenden Säuren wie Milchsäuren erfolgen. Entzuckerte und entwässerte Zuckerrübenschnitzel sind obendrein schleifkörperreich, grob und hart und erfordern somit erhebliche Kauarbeit; aufgrund ihrer Beschaffenheit erbringen sie beim Kauen gleichzeitig eine mechanische Reinigung. Zuckerrübenschnitzel fallen in erheblichem Umfang bei der Herstellung von Zucker aus Zuckerrüben an. Zu ihrer Herstellung kann somit auf die üblichen Herstellungsverfahren zurückgegriffen werden. Da übliche Zuckerrübenschnitzel einen sehr hohen Siliciumgehalt aus Sand und Erde aufweisen, werden vorzugsweise Zuckerrübenschnitzel verwendet, die aus zuvor geschälten Zuckerrüben erhalten werden. Bei der Trocknung der Schnitzel sollten nur solche Rauchgase verwendet werden, die eine Kontamination durch Sulfit und Schwermetalle verhindern. Weiterhin darf den Zuckerrübenschnitzeln keinesfalls Melasse zugesetzt werden, wie dies bei der bisherigen Verwertung von Zuckerrübenschnitzeln als Viehfutter der Fall ist.

Das erfindungsgemäße kau- und schluckbare Ballaststoffpräparat enthält die entzuckerten und entwässerten Zuckerrübenschnitzel in Dosisform. Hierzu werden sie vorzugsweise in Mengen von 0,5 g bis 3 g, vorzugsweise 1 g bis 2 g, tablettiert, pelletiert oder granuliert. Insbesondere die Tablettierung ist technisch leichter durchführbar, wenn man stärkefreie Bindemittel, beispielsweise Gelatine, einsetzt. Um das erfindungsgemäße Ballaststoffpräparat optisch und geschmacklich angenehmer zu gestalten, werden ggf. Geschmacksstoffe, Aromastoffe, zuckerfreie Süßstoffe und Farbstoffe zugesetzt. Sofern keine Geschmacks- oder Aromastoffe zugegeben werden, schmecken die erfindungsgemäßen Präparate ähnlich wie Haferflocken. Gewünschtenfalls können ferner fluorhaltige Zusätze und Desinfektionsmittel enthalten sein, welche zu einer Festigung des Zahnschmelzes führen können und eine Desinfektion der Zähne und der Mundhöhle bewirken. Der Zusatz von Desinfektionsmitteln kann weiterhin die Stabilität und Lagerfähigkeit der erfindungsgemäßen Ballaststoffpräparate erhöhen. An sich sind jedoch entzuckerte und entwässerte Zuckerrübenschnitzel auch ohne Zusatz von Desinfektionsmitteln lange Zeit haltbar und relativ unempfindlich gegen Zersetzung durch Mikroorganismen. Um den Zutritt von Feuchtigkeit zu vermindern und dadurch die Haltbarkeit zu steigern, schließlich jedoch auch um das Aussehen und die Verpackungs- und Transportmöglichkeiten zu verbessern, können die erfindungsgemäßen Ballaststoffpräparate mit einem wasserlöslichen Filmüberzug versehen oder dragiert werden. Das Dragieren sollte vorzugsweise ohne größere Mengen Stärke und Saccharose erfolgen. Da derartige Überzüge jedoch bei der praktischen Anwendung zuerst aufgelöst und mit dem Speichel verdünnt werden, sind prinzipiell auch zucker- und stärkehaltige Dragierüberzüge brauchbar.

Obwohl prinzipiell auch andere zuckerarme cellulosereiche Substanzen für die erfindungsgemäßen Ballaststoffpräparate in Frage kämen, sofern sie zuckerfrei, frei von anderen in der Mundhöhle abbaubaren Kohlehydraten wie Stärke sind, schleifkörperreich, grob, hart, wasserarm, ballaststoffreich, kaufähig, schluckbar und ungiftig sind, so sind die in großen Mengen leicht verfügbaren Zuckerrübenschnitzel für die Praxis optimal geeignet. Die erfindungsgemäßen kau- und schluckbaren Ballaststoffpräparate weisen gegenüber den bisherigen Mitteln folgende Vorteile auf:

1. Gegenüber der Reinigung der Zähne durch Zahnbürsten ist die Anwendung und Putztechnik bequem, einfach, zu jeder Zeit und an jedem Ort einsetzbar. Zusätzlich werden die Zähne und der Zahnhalteapparat gründlich mechanisch beansprucht. Es entstehen keine Schliffissuren und keine Verletzungen des Zahnfleisches durch unsachgemäße Putztechnik.

2. Mundduschen bewirken nur eine oberflächliche Reinigung und eine Massage des Zahnfleisches und lösen somit nur Teile der gestellten Aufgabe.

3. Gegenüber Kaugummi sind die erfindungsgemäßen Ballaststoffpräparate hygienischer, da sie schluckbar sind. Auch nach längerer Kaudauer entsteht kein unangenehmer Geschmack. Bereits nach kürzerer Kaudauer im Vergleich zum Kaugummi wird ein besserer Reinigungseffekt erzielt und werden Zähne und Zahnhalteapparat besser beansprucht. Die erfindungsgemäßen Ballaststoffpräparate sind auch für Magenempfindliche verträglich.

Die erfindungsgemäßen Ballaststoffpräparate sind nicht nur zur vorbeugenden Behandlung von Karies und Parodontose geeignet, sondern beugen auch einer Übersäuerung des Magens vor. Auch Dickdarmdivertikulose, Dickdarmkarzinom, Dickdarmpolypen,

- 6 -    ·    **0061710**

Cholesteringallensteine und Hypercholesterinämie werden ebenfalls vorbeugend behandelt. Dies ist durch die folgenden Effekte begründet:

Die starke Quellungsfähigkeit insbesondere durch den Pektinanteil regt die Dickdarmfunktion an. Diese Quellfähigkeit beeinflußt auch den Wasserhaushalt im Darm und wirkt einer Verhärtung der Fäzes entgegen. Aufgrund ihrer Unverdaulichkeit üben die Ballaststoffe einen mechanischen Reiz auf die Darmwand aus und regen damit die Darmmotilität an. Die Ballaststoffe bilden für die Darmflora einen wichtigen Nährboden. Beim Abbau der Ballaststoffe durch die Darmflora kommt es zur Bildung von Gärgasen, nämlich Kohlendioxid, Ammoniak, Methan und Wasserstoff. Dadurch wird der Darminhalt aufgelockert, was zu einer Vergrößerung des Stuhlvolumens und einer Transportbeschleunigung führt. Die ballastreichere Ernährung wirkt der weitverbreiteten Obstipation entgegen und macht den chronischen Mißbrauch von Abführmitteln überflüssig. Dennoch sind diese Ballaststoffe keine Abführmittel, sondern ersetzen nur den in Zivilisationsnahrung fehlenden Ballaststoffanteil. Die Ballaststoffe der Zuckerrübenschnitzel sind nicht nur quellfähig, sondern besitzen auch ausgeprägte adsorptive Eigenschaften, wodurch insbesondere auch Toxine und Karzinogene in geringerem Maße resorbiert werden und zur Leber gelangen. Sofern durch diese adsorptiven Eigenschaften geringfügige Verluste von Eiweiß und Fetten entstehen, wirkt dies der meist überreichlichen Ernährung in den Industrieländern entgegen. Durch ihren Ligninanteil können diese Ballaststoffe insbesondere Gallensäure binden, wodurch der Cholesterinspiegel beeinflußt wird. Auch der Glucosestoffwechsel wird durch verlangsamte Glucoseresorption positiv beeinflußt. Bei Personen mit subklinischem Diabetes mellitus wird eine Verbesserung der Glucosetoleranz beobachtet. Schließlich führen die erfindungsgemäßen Ballaststoffpräparate zu einem gewissen Sättigungseffekt ohne wesentliche Kalorienaufnahme und wirken der Tendenz zum Übergewicht entgegen. Ferner lassen sie sich als Kompensationssubstanz für Raucher einsetzen.

- 7 -

In den nachfolgenden Beispielen sind einige typische
Ballaststoffpräparate sowie ihre Herstellung näher erläutert.

Beispiel 1

Eingesetzt werden Zuckerrüben (Beta vulgaris), ein Zuchtprodukt aus der Runkelrübe. In je 100 g sind 15 - 20 g
Zucker (Saccharose) enthalten. Die Zuckerrüben werden
mit Wasserkanonen in Wasserkanäle geschwemmt. Von hier
aus gelangen sie zum Krautabschneider. Über eine Rübenpumpe kommen sie dann zur Waschanlage, wo sie durchgewirbelt werden. Sie werden einem Schälungsprozeß unterworfen, um auch Reste von anhaftendem (Quarz-) Sand -
in der untenstehenden Analyse als Silicium aufgeführt -
zu entfernen. Anschließend gelangen die Zuckerrüben über
einen Rübenelevator zur Schneidemaschine, wo geschnitzelte Rüben entstehen, die noch den Zucker enthalten.
Die Schnitzelgröße ist varierbar. Die Schnitzel werden
in eine Länge von 0,5 bis 2 cm und eine Dicke von 1 bis
5 mm gebracht. Die entstandene Schnitzelmaische kommt nun
in einen Extraktionsturm. Hier erfolgt das Auslaugen der
Schnitzel im Gegenstromprinzip mit Wasser. Es entstehen
einerseits der Zuckerrohsaft, andererseits unausgelaugte,
entzuckerte und nasse Rübenschnitzel mit einem Gehalt von
ca. 7 % Trockensubstanz. In der Schnitzelpresse wird nun
das Wasser von den Naßschnitzeln abgepreßt. In großen,
rotierenden Behältern werden die Schnitzel anschließend
mit $SO_2$-armen und schwermetallarmen Rauchgasen im Gegenstromverfahren getrocknet. Es wird auf einen Feuchtigkeitsgehalt von maximal 10 % getrocknet.

Die entstandenen Rübenschnitzel haben folgende Zusammensetzung: Die Angaben in % beziehen sich auf Trockensubstanz.

- 8 -

| | | |
|---|---|---|
| Saccharose | 3,67 | (Der Gehalt schwankt zwischen 0 bis 4 %) |
| Asche (Carbonate) | 3,61 | |
| Organische Nicht-saccharosestoffe einschl. Mark* | 92,72 | |

Die organischen Nichtsaccharosestoffe setzen sich wiederum zusammen aus:

| | |
|---|---|
| Mark* | 88,30 |
| Lösliche Stoffe | 4,42 |
| Stickstoffhaltige Stoffe: | |
| Eiweiß | 8,4 |
| Andere stickstoff-haltige Stoffe | 0,25 |

*Der Ausdruck Mark wird in Zuckertechnologie für die wasserunlöslichen Bestandteile der Rübe (Zellgewebe-substanz) verwendet.

Neben dem durch Hitzedenaturierung unlöslich gewordenen Eiweiß enthält das Mark in den Trockenschnitzeln:

| | | |
|---|---|---|
| Zellulose | | 23,5 |
| Lignin | | 4,2 |
| Pektin | ca. | 30 |
| Pentosane | ca. | 24 |

Der Mineralstoffgehalt beträgt umgerechnet auf einen mittleren Trockensubstanzgehalt von 90 % folgende Mittelwerte:

| a) höhere Gehalte | (g/kg) |
|---|---|
| Kalium | 5,7 |
| Natrium | 1,4 |
| Calcium | 8,3 |
| Magnesium | 2,2 |
| Phosphor | 0,9 |
| Chlor | 1,0 |

| | |
|---|---|
| Schwefel | 3,3 |
| Silicium (als Quarz) | 0,1 |

Bei ungeschälten Zuckerrüben als Ausgangsmaterial findet man Silicium (als Quarz) bis 11 g/kg.

| b) Spurenelemente | (mg/kg) |
|---|---|
| Eisen | 572 |
| Mangan | 66 |
| Kupfer | 16 |
| Kobalt | 0,39 |
| Zink | 22 |
| Molybdän | 0,43 |
| Jod | 1,8 |

Beispiel 2 (Dragees)

I. Zusammensetzung

a) Komprimat

| | |
|---|---|
| Rübenschnitzel, zerkleinert | 1403 mg |
| Pfefferminzöl | 22 mg |
| Saccharin-Natrium | 15 mg |
| Methylcellulose | 60 mg |
| | 1500 mg |

b) Dragéedecke

| | |
|---|---|
| Mannit | 500 mg |
| Methylcellulose | 50 mg |
| Calciumcarbonat | 100 mg |
| Talkum | 200 mg |
| Titandioxid | 50 mg |
| Sorbit | 100 mg |
| | 1000 mg |

Die Inhaltsstoffe des Komprimats werden gut miteinander vermischt und anschließend in einer Tablettenpresse zu Komprimaten von je 1500 mg verpreßt. Sie werden anschließend mit einer Dragee-Decke überzogen und wiegen fertig ca. 2500 mg. Sind sind längere Zeit unverändert haltbar. Diese Dragees können nach kurzer Zeit durch Einspeicheln und Kauen in einen weichen Brei umgewandelt werden. Dieser Brei kann bereits nach einigen Minuten Kauens heruntergeschluckt werden. Durch das Kauen des Breies wird der Speichelfluß stark angeregt, so daß auch nach dem Herunterschlucken noch so viel Speichel fließt, daß die verbliebenen Schnitzel leicht erfaßt und ebenfalls mit Speichel zusammen geschluckt werden können. Durch drei- bis viermaliges Kauen und Schlucken eines derartigen Dragees pro Tag wird der Fehlbedarf an Zivilisationsnahrung an Ballaststoffen voll gedeckt. Durch längeres Kauen erfolgt nachweislich eine gründliche Reinigung der Zähne und eine Festigung des Zahnfleisches.

**0061710**

P a t e n t a n s p r ü c h e

1. Kau- und schluckbares Ballaststoffpräparat zur Zahnpflege und Ergänzung der Nahrung, dadurch gekennzeichnet,
   daß es überwiegend aus entzuckerten und entwässerten
   Zuckerrübenschnitzeln in Dosisform besteht und gegebenenfalls stärkefreie Bindemittel, Geschmacksstoffe, Aromastoffe, zuckerfreie Süßstoffe, fluorhaltige Zusätze,
   Desinfektionsmittel und Farbstoffe enthält.

2. Ballaststoffpräparat gemäß Anspruch 1 , dadurch gekennzeichnet, daß es tablettiert, pelletiert oder granuliert ist und pro
   Dosis 0,5 g bis 3,0 g, vorzugsweise 1 g bis 2 g Zuckerrübenschnitzel enthält.

3. Ballaststoffpräparat gemäß Anspruch 1  oder 2 , dadurch
   gekennzeichnet,daß es mit einem wasserlöslichen Filmüberzug versehen oder dragiert ist.

4. Ballaststoffpräparat gemäß einem der Ansprüche 1  bis 3 ,
   dadurch gekennzeichnet, daß es Zuckerrübenschnitzel aus
   zuvor geschälten Zuckerrüben enthält.

5. Verfahren zur Herstellung eines Kau- und schluckbaren
   Ballaststoffpräparats zur Zahnpflege und Ergänzung der
   Nahrung, dadurch gekennzeichnet, daß man entzuckerte und
   entwässerte Zuckerrübenschnitzel in Portionen von 0,5 g
   bis 3,0 g, vorzugsweise 1 g bis 2 g, gewünschtenfalls
   zusammen mit stärkefreien Bindemitteln, Geschmacksstoffen,
   Aromastoffen, zuckerfreien Süßstoffen, fluorhaltigen
   Zusätzen, Desinfektionsmitteln und Farbstoffen tablettiert,
   pelletiert oder granuliert und gewünschtenfalls mit einem
   wasserlöslichen Filmüberzug versieht oder dragiert.

6. Verfahren gemäß Anspruch 5 , dadurch gekennzeichnet, daß
   es aus Zuckerrübenschnitzeln zuvor geschälter Zuckerrüben
   hergestellt ist.

- 12 -

7. Verwendung eines Ballaststoffpräparats gemäß einem der
   Ansprüche 1 bis 4 zur vorbeugenden Behandlung von Karies
   und Parodontopathien und Erkrankungen des Gastointestinaltrakts.